# Europäisches Patentamt
# European Patent Office
# Office européen des brevets

(11) Publication number: **0 105 706**
**B1**

(12) **EUROPEAN PATENT SPECIFICATION**

(45) Date of publication of patent specification: **19.08.87**

(51) Int. Cl.⁴: **G 02 B 6/10, A 61 B 17/36**

(21) Application number: **83305818.3**

(22) Date of filing: **28.09.83**

(54) Flexible dielectric waveguide for high efficiency middle IR wavelength transmission.

(30) Priority: **29.09.82 US 427076**

(43) Date of publication of application:
**18.04.84 Bulletin 84/16**

(45) Publication of the grant of the patent:
**19.08.87 Bulletin 87/34**

(84) Designated Contracting States:
**DE FR GB**

(56) References cited:     -
**US-A-3 414 344**
**US-A-3 995 934**
**US-A-4 112 028**

(73) Proprietor: **LAAKMANN ELECTRO-OPTICS INC.**
**33052 Calle Aviador, Suite B**
**San Juan Capistrano California 92675 (US)**

(72) Inventor: **Laakmann, Katherine Dexter**
**23821 Daysa Circle**
**Laguna Niguel California (US)**
Inventor: **Laakmann, Peter**
**23821 Daysa Circle**
**Laguna Niguel California (US)**

(74) Representative: **Colgan, Stephen James et al**
**CARPMAELS & RANSFORD 43 Bloomsbury**
**Square**
**London WC1A 2RA. (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

This invention relates to dielectric waveguides and more particularly to a flexible optical fiber for guiding infrared (IR) laser light generated by a gas laser, typically a CO or a $CO_2$ laser.

Flexible waveguides, frequently referred to as optical fibers, for the transmission of optical signals are used widely now in the field of optical communications. The transmission of infrared laser light through a flexible optical waveguide has tremendous appeal in both industrial and medical applications.

The typical infrared optical fiber for the transmission of $CO_2$ laser power utilizes a polycrystalline cladded fiber. One such fiber is Kristen/5 developed by Horiba, Ltd. of Kyoto, Japan, described in Horiba Bulletin: HRE-3827A. Kristen/5 is an extruded bulk fiber of thallium bromoiodide (KRS-5), an alkali halide. While Horiba's Kristen/5 waveguide provides high power transmission of $CO_2$ laser energy at 10.6 μm there are substantial radiation losses noted with this material due to reflection at the end surfaces of the fiber as well as internal absorption and scattering inside and along the fiber surfaces, resulting in the product having a total transmissivity rating for the middle infrared wavelengths of approximately 55%. To offset these losses a more powerful source of $CO_2$ laser energy is required. Such increases in $CO_2$ laser power output result in approximately proportional increases in laser generating cost.

A KRS-5 fiber is extremely flexible at room temperature as reported by D. A. Pinnow et al. of the Hughes Research Laboratories in Malibu, California, in "Polycrystalline fiber optical waveguide for infrared transmission", Appl. Phys. Lett. 33(1), 1 July 1978 at p. 28. Horiba has found, however, that Kristen/5 deteriorates when exposed for many hours at temperatures in excess of 80°C. To prevent overheating, the Horiba fibre is air cooled through an outer cylinder sleeve. Thus, even though the crystalline core has a diameter in the 1 mm range, the completed Horiba infrared cable has a relatively large outer diameter making it impractical for endoscopic applications. In addition, KRS-5 is toxic and dangerous when ingested. See "Guide to ir-transmissive materials", Laser Focus, December 1976 at p. 48.

US Patent No. 3995934 describes a flexible light guide but the core is entirely liquid and therefore gives poor transmission of the light.

It would be desirable to provide a flexible optical waveguide for high power high efficiency transmission of $CO_2$ laser energy at 10.6 μm which is sufficiently slim to conveniently pass through the throat of a normal human patient.

According to the present invention, a flexible dielectric waveguide as defined in claim 1 is provided.

The waveguide of the invention is all dielectric and requires no air cooling. Said waveguide comprises a conventional cylindrical or square cross-sectional cladding surrounding an infrared light transmitting core with the core index of refraction being greater than that of the cladding.

In a preferred embodiment, the solid rods are made of zinc selenide, ZnSe, an amorphous Irtran material of hotpressed polycrystalline composition commercially available from the Eastman Kodak Company. ZnSe is an excellent transmitter of laser light at middle infrared wavelengths; is not hygroscopic, thus its transmissivity will not degrade with time (water absorbs IR laser light); and is also a non-toxic material. Continuous transmission as well as the requisite overall flexibility to the waveguide is accomplished by limiting the solid inflexible ZnSe rods to approximately one centimeter lengths and immersing them in a liquid dielectric such as carbon disulfide, $CS_2$. Since there is relatively little liquid in the core, the transmissive efficiency of the liquid at the requisite wavelength need not be as great as that of the solid dielectric. Since the index of refraction of ZnSe is substantially different than that of $CS_2$, the ZnSe rods are coated to minimize the reflection losses that would otherwise occur at the interfaces between the two dielectrics of the different refractive indices.

In another preferred embodiment, the solid rods are again made of a highly IR transmissive material with an absorption coefficient less than 0.005 $cm^{-1}$, in the range of about 0.001 $cm^{-1}$, such as cesium iodide, CsI, which has an index of refraction, $\eta_r$ of about 1.72. The liquid in this embodiment is a combination of two miscible liquids, one having a refractive index greater than $\eta_r$ and the other less than $\eta_r$. The two liquids are mixed in such proportion that their combined refractive index matches $\eta_r$, that of the solid rods. This embodiment obviates the need to coat the rods.

In the best mode of our flexible dielectric waveguide, a one meter length having a single 90 degree bend will transmit in excess of 90 percent of high power $CO_2$ laser energy at the prescribed middle infrared wavelength of 10.6 μm. The effects of bends is presented in quantitative terms in a separate section below.

Fig. 1 shows in perspective an infrared optical waveguide cable according to the present invention;

Fig. 2 shows an enlarged section taken along line 2—2 of Fig. 1 illustrating one of the solid rods used for transmitting infrared energy;

Fig. 3 is a section taken along line 3—3 of Fig. 2 wherein the solid rod has a square cross-section;

Fig. 4 is a fragmentary elevation of a portion of the cable;

Fig. 5 is an enlarged section of a portion of the cable similar to Fig. 2 in a bent orientation;

Fig. 6a illustrates a handpiece having a lens for focussing the laser output;

Fig. 6b illustrates a tapered hollow dielectric pipe for use as an end piece;

Fig. 6c illustrates a tapered solid dielectric end piece;

Fig. 7 is a section similar to Fig. 3 with an alternate configuration for the cladding; and

Fig. 8 is a view similar to Fig. 3 illustrating an alternate embodiment wherein the solid rod has a circular cross-section.

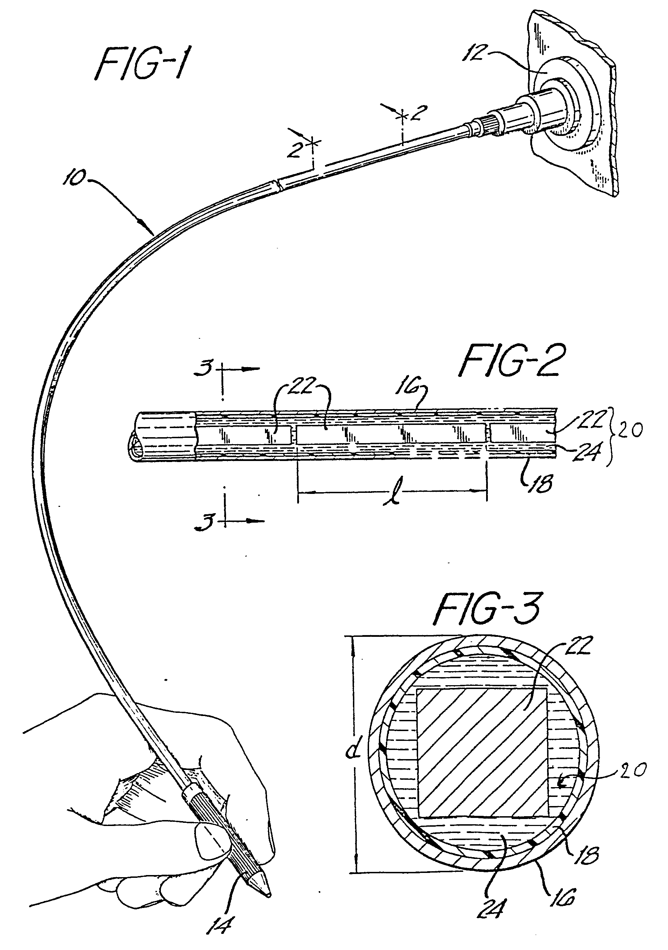

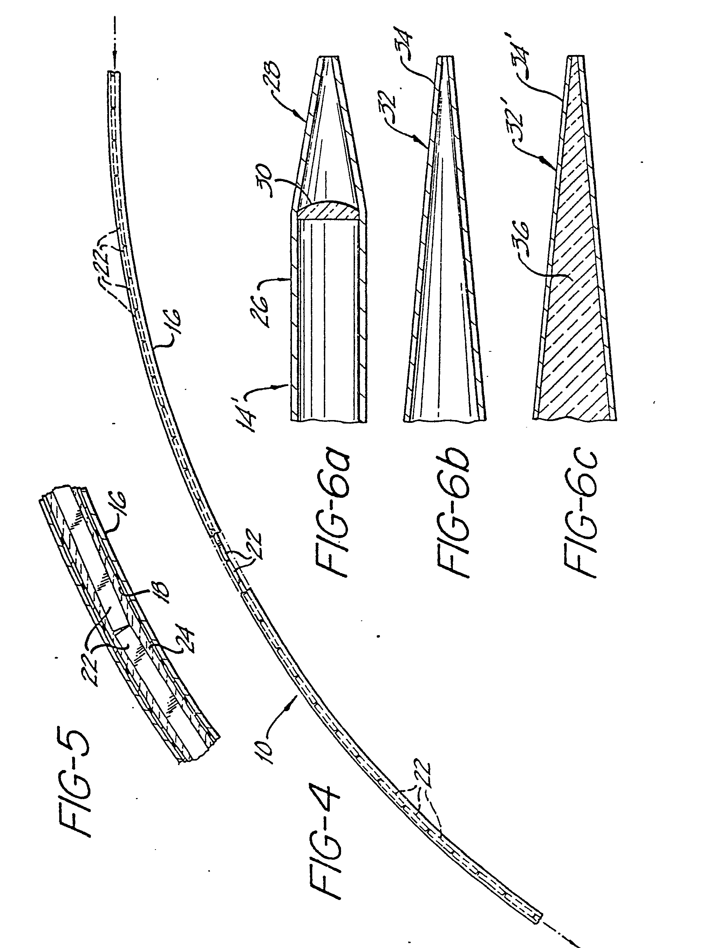

An infrared "optical fiber", which is a certain type of dielectric waveguide for guiding light waves, designated generally as 10, is illustrated in Fig. 1. The optical fiber cable 10 is shown attached at one end to a source of laser energy by means of an interface 12 as shown in partial schematic in Fig. 1. Interface 12 designates the input end of the optical fiber 10 and typically includes a lens (not shown) to match the incoming laser beam to the fiber 10. Fig. 1 also shows in schematic form a handpiece 14, to permit manipulation of the laser output. One or more lenses may be included in the handpiece 14 to provide collimation of the light output emanating from the optical fiber. The fiber 10 may also end in any of a variety of end pieces examples of which are illustrated in Figs. 6a—6c and discussed below.

For purposes of this discussion, the optical fiber 10 will refer to the entire cable between interface 12 and handpiece 14 and not merely to the waveguide portion. A detailed straight portion section of the optical fiber 10 variously referred to as a "light pipe" or a flexible dielectric waveguide is shown in Fig. 2. In a preferred embodiment, the optical fiber 10 is approximately a one meter long cylindrical cable. The fiber 10 includes a flexible outer sleeve 16 having good thermal conductivity as well as durability and is preferably fabricated of braided metal stands. On the inner surface of the outer sleeve 16 there is provided a concentric cladding material 18 having a relatively low index of refraction such as a plastic (e.g., Teflon*) to thereby avoid scatter and field distortion of the transmitted light. The cylindrical region within the cladding material 18 is generally referred to as the core 20 in conventional optical fiber terminology. The materials comprising the core are selected for their high index of refraction relative to that of the cladding material 18 to afford total internal reflection within core 20 of the transmitted laser light. The cladding material need not be IR transmissive as discussed in detail below.

In the best mode of the present invention, the core 20 is all dielectric and includes a plurality of solid rods 22 suspended in a liquid medium 24. The rods have a length l of approximately one centimeter and are fabricated from a compound having a very high rate of transmission of infrared energy at a wavelength of 10.6 micrometers, the wavelength characteristic of $CO_2$ lasers. One such material is the non-hygroscopic dielectric Irtran material zinc selenide (Irtran 4), which has a refractive index of approximately 2.4. ZnSe has total transmission loss at 10.6 μm of less than 0.1% per centimeter. There are a few materials that may be substituted for ZnSe, such as certain alkali halides, for example, polvcrystalline thallium bromide and thallium bromoiodide (KRS-5). However, such halides require a vacuum tight environment because of their propensity to absorb moisture from the atmosphere which in turn deteriorates their IR transmissivity. A non-exhaustive list of IR transmissive materials for rods is set forth in Table 1.

TABLE 1

| Material | Index of refraction* | Absorption coefficient/cm* |
|---|---|---|
| ZnSe | 2.40 | 0.001 |
| NaCl | 1.49 | 0.002 |
| KCl | 1.45 | 0.001 |
| AgCl | 1.98 | 0.005 |
| AgBr | 2.00 | 0.005 |
| Ge | 4.01 | 0.032 |
| KRS-5 | 2.38 | 0.001 |
| Diamond | 2.00 | 0.0003 |
| CdTe | 2.71 | 0.002 |
| GaAs | 3.76 | 0.01 |
| CsI | 1.72 | |
| KBr | 1.53 | 0.0004 |

* Approximate.

* Registered Trademark of E. I. du Pont de Nemours & Co.

3

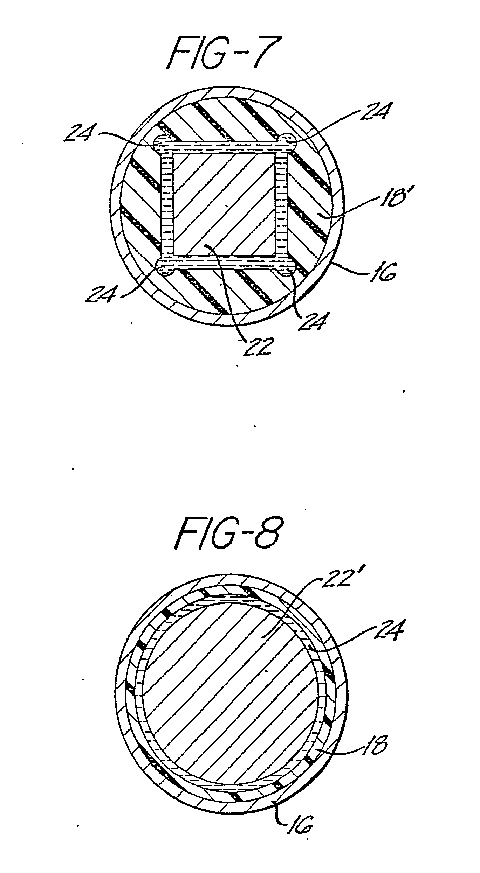

As shown in Fig. 3, the solid rod 22 has a square cross-section approximately 1 mm×1 mm with the diagonal dimension of the square being slightly less than the inside diameter of the cladding material 18. For ease of fabrication and to minimize the amount of liquid, an alternate cladding material configuration is used as shown in Fig. 7. The cladding material 18' has a cylindrical exterior but a modified interior, conforming approximately to the square shape of the rods 22. Alternatively, the rods have a circular cross-section as illustrated by rod 22' in Fig. 8. If a circular cross-section is used, the diameter of rod 22' is somewhat less than the inside diameter of the cladding material 18.

The solid rods 22 are suspended in a liquid dielectric material 24, preferably carbon disulfide, $CS_2$, which also has high transmissivity of infrared light at a wavelength of 10.6 μm. Ether or germanium tetrachloride, $GeCl_4$, may be substituted for $CS_2$ as the liquid 24. Regardless of the choice of liquid, however, it must have a high rate of transmissivity of infrared energy at the prescribed wavelength and since the cable 10 is intended to be inserted into a patient's throat, it should preferably be relatively non-toxic. For use as an endoscope, the outer diameter, d, of the cable 10 is about 2 mm.

$CS_2$ has a refractive index of approximately 1.6. In the ideal situation, the refractive index of the liquid 24 should be the same as the refractive index of the solid rods 22. Otherwise, the propagation of the light wave through the dielectric interface between the two materials will result in a portion of the wave being reflected at the interface, thereby resulting in attenuation of the transmission. This problem is avoided by index matching between the liquid 24 and the solid rods 22 by coating the solid rods with a material having a third index of refraction. The coating should be one-fourth the thickness of the wavelength of the transmitted wave and should have an index of refraction satisfying the following relationship:

$$\eta_c = (\eta_1 \cdot \eta_r)^{1/2}, \tag{1}$$

where:

$\eta_c$=index of refraction of the coating material;

$\eta_1$=index of refraction of the liquid; and

$\eta_r$=index of refraction of the rod.

Thus, for a ZnSe rod having an index of refraction of approximately 2.4 disposed in liquid $CS_2$ having an index of refraction of approximately 1.6, the index of refraction of the coating material should be in the range of 1.9 to 2.0. The coating may be applied to the ZnSe rods by the well-known vacuum deposition technique.

The requirement for coating the solid rods 22 is obviated by suspending the rods 22 in a combination of two or more IR transmissive miscible liquids in such proportion that the index of refraction of the combination matches the refractive index of the material used for the rods.

Preferred choices for these liquids are carbon diselenide, $CSe_2$, which has a refractive index of about 1.85, combined with carbon disulfide, $CS_2$, which has a refractive index of about 1.63. A preferred choice for the rod material then would be an IR transmissive solid having a refractive index within the range for these miscible liquids, such as cesium iodide, CsI, which has a refractive index of about 1.72. Other combinations of IR transmissive miscible liquids will permit the choice of other rod materials. Since most IR transmissive liquids have refractive indices lower than that of ZnSe, the choice of solid rod materials utilizing this technique is limited. In general, most low loss IR transmissive materials are compounds having a relatively simple molecular configuration comprising heavy atoms thereby assuring fewer vibrational quantum numbers.

Still another embodiment to the liquid/solid light pipe is one in which the liquid is not necessarily a liquid at room temperature, but has a low melting and/or softening temperature (<100°C). The melting or softening temperature is maintained by the use of heater elements around the light pipe. Of particular utility, for the 10.6 μm region is selenium, which is relatively non-toxic in elemental form and which has excellent IR transmission. As a further advantage, selenium's refractive index is very close to ZnSe's refractive index, so that uncoated ZnSe rods serve as the solid rod material. Finally, molten selenium is highly compatible chemically with ZnSe.

As assembled, the fiber 10 will include sufficient numbers of the solid rods 22, as shown in Fig. 4, so that, on the average, the separation between adjacent rods 22 will be on the order of 0.01 to 0.05 millimeter. Such spacing, see Fig. 5, will permit a one meter length of fiber 10 to be able to take a 90 degree bend. The total liquid path length is approximately that of the diameter d of the fiber, i.e., about 1—5 mm. Since the absorption coefficient α of the liquid is much greater than that of the rods, the amount of the liquid should be minimized as constrained by the 90° bending requirement. When ZnSe is used as the material for the rods 22 and the liquid medium 24 is $CS_2$, a one meter length of fiber 10 even when deflected 90 degrees will transmit approximately 90 percent or more of the 10.6 μm infrared light it receives from a carbon dioxide laser.

For ease of fabrication and to minimize the volume of liquid, an alternate cladding material configuration as shown in Fig. 7 is used. The cladding material 18' has a cylindrical exterior but a modified interior, conforming approximately to the square shape of the rods 22.

Any of several end pieces, such as handpiece 14 or the embodiments illustrated in Figs. 6a, 6b, and 6c, may be conveniently attached in known fashion to the end of the light pipe 10 for directing the output as needed. The handpiece 14' is illustrated in Fig. 6a includes a cylindrical portion 26 whose outer diameter matches that of the light pipe 10. Forward of the cylindrical portion 26 there is a tapered portion 28 from which the beam exists. A lens 30 is provided to collimate the exiting output laser beam. An alternative to handpiece 14' is a tapered hollow endpiece 32. The endpiece 32 has a solid outer sleeve 34 which is connectable to the output end of light pipe 10. Otherwise, the endpiece 32 is hollow with collimation achieved by its tapered configuration. The same configuration as that of endpiece 32 is disclosed in Fig. 6c in which the endpiece 32' also has an outer sleeve 34' but is further provided with a core 36. Preferably the core is made of selenium glass; however, in general, any dielectric material will function provided its refractive index is greater than that of the outer sleeve 34'.

Effects of bends

The decollimation experienced for a 90° bend is given by:

$$\Delta \cos \alpha_{out} = \frac{2dR \cos \alpha_{in}}{R^2 - (d/2)^2} \qquad (2)$$

where:

d = fiber diameter;
R = bend radius;
$\alpha_{in}$ = half angle of incident cone; and
$\alpha_{out}$ = half angle of emergent cone.
For d≪R, this reduces to:

$$\cos \alpha_{out} = \cos \alpha_{in}[1 - 2d/R] \qquad (3)$$

For total internal reflection, the critical incident angle $\theta_c$ is given by:

$$\frac{\eta_1 \sin \theta_c}{\eta_2} > 1 \qquad (4)$$

where:

$\eta_1$ = core index; and
$\eta_2$ = cladding index.
Combining (3) and (4):

$$\frac{\eta_1}{\eta_2} > \frac{1}{\cos \alpha_{in}[1 - 2d/R]} \qquad (5)$$

For multiple bends, it follows that the half angle of the emergent cone is given by:

$$\cos \alpha_{out} = [1 - 2d/R]^\eta \qquad (6)$$

where $\eta$ is the number of 90° bends. Similarly, the refractive index, for perfect initial collimation (cos $\alpha_{in}$ = 1), must obey:

$$\frac{\eta_1}{\eta_2} \geq \frac{1}{[1 - 2d/R]^\eta} \qquad (7)$$

Cladding materials

Various cladding materials are possible for this light pipe.
Defining:

$$\eta_2 = \eta_{2R} + i\eta_{2i} \qquad (8)$$

where:

$\eta_2$ = the complex refractive index of cladding;
$\eta_{2R}$ = the real part of the refractive index of cladding;
$\eta_{2i}$ = the imaginary part of the refractive index of cladding; and
$\eta_1$ = the refractive index of core.

From the previous derivation, it was shown that

$$\frac{\eta_1}{\eta_2} \geq \frac{1}{[1-2d/R]^\eta} \tag{7}$$

for:

$\eta=2$
$d=1$ mm
$R=10$ cm, equation (7) yields:

$$\frac{\eta_1}{\eta_{2R}} \geq 1.04$$

Similarly, in order for the modal analysis to be valid in a dense dielectric guide, it can be shown that:

$$2\,\eta_{2i}\,\eta_{2R} \ll \eta_1{}^2 - \eta_{2R}{}^2 \tag{8}$$

or

$$\eta_{2i} \ll (\eta_i{}^2 - \eta_{2R}{}^2)/2\eta_{2R} \tag{9}$$

For

$$\eta_1 = 1.6$$

and

$$\eta_2 = 1.5$$

$$\eta_{2i} \ll .1$$

If

$$\eta_{2i} \leq .01,$$

$\alpha < 120$ cm$^{-1}$, where $\alpha = -dl/dz/l =$ absorption coefficient.

Hence, a material which is essentially opaque in the IR can still be suitable as a cladding material. Certainly, most plastics have absorption coefficients less than 120 cm$^{-1}$.

## Claims

1. A flexible dielectric waveguide (10) for use with a $CO_2$ laser to transmit infrared light at a prescribed wavelength of 10.6 µm, which comprises an infrared light transmitting core (20), surrounded by a cladding (18, 18') of index of refraction lower than that of the core, characterized in that:

a) said cladding (18, 18') is encased in a flexible cylindrical sleeve (16) having an outer diameter not substantially greater than 2 millimeters; and

b) said infrared transmitting core (20) comprises: an infrared transmissive liquid (24) disposed within said cladding and having a first refractive index, said liquid (24) having a transmission loss of infrared energy at the prescribed wavelength not substantially greater than 10.0 percent per millimeter; and

a plurality of elongated solid rods (22, 22') having a second refractive index and disposed axially, end to end within said cladding in a closely packed arrangement allowing for some motion within said liquid, said rods (22, 22') having a transmission loss of infrared energy at the prescribed wavelength no more than 0.5 percent per centimeter of length, said second refractive index at the prescribed wavelength being no less than said first refractive index.

2. A dielectric waveguide according to claim 1 wherein said second index of refraction is higher than said first index of refraction and further comprising means for providing for close to zero reflection of infrared light between said liquid (24) and said rods (22, 22').

3. A dielectric waveguide according to claim 2 wherein said solid rods (22, 22') are coated with a material having a third index of refraction which is approximately the square root of the product of the first and second indices.

4. A dielectric waveguide according to claim 1 wherein said infrared transmissive liquid (24) comprises a mixture of two miscible components of different refractive indices and combined in such proportion that their combined refractive index matches that of the elongated solid rods (22, 22').

5. A dielectric waveguide according to claim 4 wherein said rods (22, 22') are of CsI and the infrared transmissive liquid (24) comprises a mixture of $CSe_2$ and $CS_2$.

6. A dielectric waveguide according to any of claims 1 to 3 wherein said flexible cylindrical sleeve (16) provided with said cladding (18, 18') surrounds a substantially non-hygroscopic dielectric core (20).

7. A dielectric waveguide according to claim 6 wherein said solid rods (22, 22') are of ZnSe.

8. A dielectric waveguide according to claim 7 wherein said infrared transmissive liquid (24) is $CS_2$, ether, $GeCl_4$, or molten selenium.

9. A dielectric waveguide according to any of claims 1 to 8 wherein the constitutive elements of said

**0 105 706**

waveguide are dimensioned in a manner such that a meter length section of said waveguide may be deflected 90 degrees.

10. A dielectric waveguide according to any of claims 1 to 9 wherein said infrared transmissive liquid (24) is relatively non-toxic.

## Patentansprüche

1. Flexibler dielektrischer Wellenleiter (10) zur Verwendung mit einem $CO_2$-Laser zur Übertragung von Infrarotlicht einer vorgeschriebenen Wellenlänge von 10,6 µm, enthaltend einen Infrarotlichtübertragungskern (20), der von einer Umhüllung (18, 18') mit niedrigerem Brechungsindex als der des Kerns umgeben ist, dadurch gekennzeichnet, daß:

a) die Umhüllung (18, 18') von einer flexiblen, zylindrischen Hülse (16) umgeben ist, die einen Außendurchmesser hat, der nicht wesentlich größer als 2 mm ist, und

b) der Infrarotübertragungskern (20) enthält: eine infrarotübertragende Flüssigkeit (24), die in der Umhüllung angeordnet ist und einen ersten Brechungsindex hat, welche Flüssigkeit (24) einen übertragungsverlust für Infrarotenergie bei der vorgeschriebenen Wellenlänge von nicht wesentlich größer als 10% pro Millimeter aufweist; und mehrere langgestreckte massive Stäbe (22, 22'), die einen zweiten Brechungsindex haben und axial, Ende an Ende in der Umhüllung in einer dichtgepackten Anordnung, die eine gewisse Bewegung innerhalb der Flüssigkeit erlaubt, angeordnet sind, wobei die Stäbe (22, 22') einen übertragungsverlust für Infrarotenergie bei der vorgeschriebenen Wellenlänge von nicht mehr als 0,5% pro Längenzentimeter aufweisen und der zweite Brechungsindex bei der vorgeschriebenen Wellenlänge nicht kleiner als der erste Brechungsindex ist.

2. Dielektrischer Wellenleiter nach Anspruch 1, bei dem der zweite Brechungsindex größer als der erste Brechungsindex ist, und weiterhin enthaltend eine Einrichtung zum Erzeugen einer Nahezu-Null-Reflexion von Infrarotlicht zwischen der Flüssigkeit (24) und den Stäben (22, 22').

3. Dielektrischer Wellenleiter nach Anspruch 2, bei dem die massiven Stäbe (22, 22') mit einem Material beschichtet sind, das einen dritten Brechungsindex hat, der etwa die Quadratwurzel des Produktes aus den ersten und zweiten Brechungsindizes ist.

4. Dielektrischer Wellenleiter nach Anspruch 1, bei dem die infrarotübertragende Flüssigkeit (24) aus einer Mischung aus zwei mischbaren Komponenten unterschiedlicher Brechungsindizes besteht, die in einem solchen Verhältnis kombiniert sind, daß ihr kombinierter Brechungsindex mit dem der langgestreckten massiven Stäbe (22, 22') übereinstimmt.

5. Dielektrischer Wellenleiter nach Anspruch 4, bei dem die Stäbe (22, 22') aus CsI bestehen und die infrarotübertragende Flüssigkeit (24) aus einer Mischung aus $CSe_2$ und $CS_2$ besteht.

6. Dielektrischer Wellenleiter nach einem der Ansprüche 1 bis 3, bei dem die flexible zylindrische Hülse (16), die mit der Umhüllung (18, 18') versehen ist, einen im wesentlichen nicht-hygroskopischen dielektrischen Kern (20) umgibt.

7. Dielektrischer Wellenleiter nach Anspruch 6, bei dem die massiven Stäbe (22, 22') aus ZnSe bestehen.

8. Dielektrischer Wellenleiter nach Anspruch 7, bei dem die infrarotübertragende Flüssigkeit (24) $CS_2$, Äther, $GeCl_4$ oder geschmolzenes Selen ist.

9. Dielektrischer Wellenleiter nach einem der Ansprüche 1 bis 8, bei dem die Bestandteile des Wellenleiters derart dimensioniert sind, daß ein Längenabschnitt von einem Meter des Wellenleiters um 90° gebogen werden kann.

10. Dielektrischer Wellenleiter nach einem der Ansprüche 1 bis 9, bei dem die infrarotübertragende Flüssigkeit (24) relativ ungiftig ist.

## Revendications

1. Guide d'onde diélectrique flexible (10) destiné à être utilisé avec un laser à $CO_2$ pour transmettre de la lumière infrarouge à une longueur d'onde prescrite de 10,6 µm, qui comprend un coeur transmettant la lumière infrarouge (20) entouré d'un habillage (18, 18') d'indice de réfraction inférieur à celui du coeur, caractérisé en ce que:

a) l'habillage (18, 18') est logé dans un manchon cylindrique flexible (16) ayant un diamètre externe sensiblement non supérieur à 2 mm; et

b) le coeur transmettant l'infrarouge (20) comprend:

un liquide transmissif dans l'infrarouge (24) disposé dans l'habillage et ayant un premier indice de réfraction, ce liquide (24) présentant une perte par transmission d'énergie infrarouge à la longueur d'onde prescrite sensiblement non supérieure à 10,0% par mm; et

une pluralité des bâtonnets solides allongés (22, 22') ayant un second indice de réfraction et disposés axialement bout à bout dans l'habillage selon un agencement étroitement serré permettant un certain déplacement dans le liquide, les bâtonnets (22, 22') présentant une perte de transmission dans l'infrarouge à la longueur d'onde prescrite non supérieure à 0,5% par cm de longueur, le second indice de réfraction à la longueur d'onde prescrite n'étant pas inférieur au premier indice de réfraction.

7

2. Guide d'onde diélectrique selon la revendication 1, dans lequel le second indice de réfraction est supérieur au premier indice de réfraction et comprenant en outre des moyens pour assurer une réflexion de la lumière infrarouge proche de zéro entre le liquide (24) et les bâtonnets (22, 22').

3. Guide d'onde diélectrique selon la revendication 2, dans lequel les bâtonnets solides (22, 22') sont revêtus d'un matériau présentant un troisième indice de réfraction qui est approximativement la racine carrée du produit des premier et second indices.

4. Guide d'onde diélectrique selon la revendication 1, dans lequel le liquide transmissif à infrarouge (24) comprend un mélange de deux composants miscibles d'indices de réfraction différents et combinés dans une proportion telle que leur indice de réfraction combiné s'adapte à celui des bâtonnets solides allongés (22, 22').

5. Guide d'onde diélectrique selon la revendication 4, dans lequel les bâtonnets (22, 22') sont en CsI et le liquide transmissif dans l'infrarouge (24) comprend un mélange de $CSe_2$ et de $CS_2$.

6. Guide d'onde diélectrique selon l'une quelconque des revendications 1 à 3, dans lequel le manchon cylindrique flexible (16) muni de l'habillage (18, 18') entoure un coeur diélectrique pratiquement non hygroscopique (20).

7. Guide d'onde diélectrique selon la revendication 6, dans lequel les bâtonnets (22, 22') sont en ZnSe.

8. Guide d'onde diélectrique selon la revendication 7, dans lequel le liquide transmissif dans l'infrarouge (24) est $CS_2$, de l'éther, $GeCl_4$ ou du sélénium fondu.

9. Guide d'onde diélectrique selon l'une quelconque des revendications 1 à 8, dans lequel les éléments constitutifs du guide d'onde ont des dimensions telles qu'un morceau du guide d'onde d'une longueur de un métre peut être défléchi de 90°.

10. Guide d'onde diélectrique selon l'une quelconque des revendications 1 à 9, dans lequel le liquide transmissif dans l'infrarouge (24) est relativement non toxique.

0 105 706

*FIG-1*

*FIG-2*

*FIG-3*

1

FIG-5

22
16
18
24

FIG-4

22
16
22
10
22

0 105 706

FIG-6a

14'
26
30
28

FIG-6b

32
34

FIG-6c

36
32'
34'

## FIG-7

## FIG-8